(19) Europäisches Patentamt
European Patent Office
Office européen des brevets

(11) Publication number: **0 203 386 B1**

(12) **EUROPEAN PATENT SPECIFICATION**

(45) Date of publication of patent specification: **13.11.91** (51) Int. Cl.5: **A61K 35/78**

(21) Application number: **86105750.3**

(22) Date of filing: **25.04.86**

(54) **Pharmaceutical composition for treating nonlymphatic leukemia and method of producing the same and its components.**

(30) Priority: **28.05.85 US 737949**

(43) Date of publication of application:
**03.12.86 Bulletin 86/49**

(45) Publication of the grant of the patent:
**13.11.91 Bulletin 91/46**

(84) Designated Contracting States:
**AT BE CH DE FR GB IT LI LU NL SE**

(56) References cited:
**GB-A- 2 035 082**

**Deutsche Apothekerzeitung: 116, 1976, p.4**

**Encyclopedia of Terpenoids: 1982, pp 1089-95**

(73) Proprietor: **Liu, Yaguang**
**67-08 168th Street**
**Fresh Meadows, NY 11365(US)**

(72) Inventor: **Liu, Yaguang**
**67-08 168th Street**
**Fresh Meadows, NY 11365(US)**

(74) Representative: **Diehl, Hermann O. Th., Dr. et al**
**Diehl & Partner Flüggenstrasse 13**
**W-8000 München 19(DE)**

**EP 0 203 386 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a new pharmaceutical composition for treating leukemia, more specifically, for treating nonlymphatic leukemia, such as acute chronic granulocytic and monocytic leukemia without adverse side effects.

Numerous antileukemia drugs have been investigated but so far, there is no single drug that is effective and safe. As discussed in U.S. 3,497,593, an alkaloid from Tylophora plant is said to have antitumor activity against mouse leukemia (L-1210). U.S. 3,928,584 discloses an organic composition derived from tree sap and is said to have activity against mouse leukemia P-388. Also U.S. 4,431,639 discloses that an extract of Rhisoma Stractylis promotes the production of lymphocytes in the circulating blood, consequently eliminating cancer growth.

Harringtonine or Homoharringtonine, hereinafter referred to as HH, has been known to be effective against acute chronic granulocytic and monocytic leukemia (Journal of Chinese Internal Medicine 3:162-164, 1978). However, it is highly toxic and causes damage to heart and hematopoietic organs. The results of experiments in animals, such as mice, rabbits and dogs, indicate that most of them die from cardiotoxicity after receiving the drug. Therefore, there is a need to improve the HH drug for safe use against leukemia. This drug is of special importance in that all known antileukemia drugs are effective against lymphatic leukemia and there are no effective drugs for treating nonlymphatic leukemia.

This object is solved by the pharmaceutical composition according to claim 1. Further advantageous features are evident from the dependent claims. The invention also provides methods for producing the composition.

The pharmaceutical composition in accordance with the present invention treats nonlymphatic leukemia, especially acute chronic granulocytic and monocytic leukemia, without side effect.

The new improved pharmaceutical composition in accordance with the present invention is named SAL in the context of this application (for Safe Anti-Leukemia). It comprises five major ingredients, namely Harringtonine, Homoharringtonine, Anethole, Oleanolic Acid and Ginsenosides.

It has been found that by addition of Anethole, Oleanolic Acid and Ginsenosides to the HH, a new composition is obtained which is effective in the treatment of nonlymphatic leukemia and which does not have the adverse side effects of HH.

The invention will be described with each component, as to its production, chemical structure and properties.

Throughout the specification, the concentration of the solvent is the same as first given unless stated otherwise. Redeuced pressure means about 2,27 kPa (17 mm Hg. abs), l is liter, kg is kilogram. ml is milliliter. Yield in weight %.

Example 1. HH is extracted from the skins, stems, leaves and seeds of Cephalotaxus fortunel Hook and other related species, such as Cephalotaxus sinensis Li, C. hainanensis, and C. wilsoniana, including C.oliveri mast and C.harringtonia.

1 kg of finely ground Cephalotaxus fortunel Hook is extracted with 8 l of 90% ethanol at room temperature for 24 hrs. The solution is filtered to yield a filtrate A and filtercake. The filtercake is percolated with ethanol and filtered again to yield filtrate B. A and B are combined and distilled under reduced pressure to recover ethanol and an aqueous residue. To this residue, 2% HCl is added to adjust the pH to 2.5. The solids are separated from the solution by filtration to yield a filtrate C. The solids are washed once with 2% HCl and filtered to yield a filtrate D. C and D are combined and the pH adjusted to 9.5 by adding saturated sodium carbonate solution. The alkaline filtrate is extracted with chloroform and the chloroform layer separated from the aqueous layer. This extration process is repeated five times. All the chloroform extracts are combined and distilled at reduced pressure to recover chloroform and alkaloid as a solid residue respectively.

The solid alkaloid is then dissolved in 20 ml. of 6% citric acid in water. The solution is divided into three equal portions. These are adjusted to pH 7,8 and 9 by adding saturated sodium carbonate solution.

The portions having pH 8 and 9 are combined and extracted with chloroform. The chloroform extracts are distilled under reduced pressure, whereby chloroform is removed and recovered and a solid residue of crude Harringtonine is obtained.

The crude Harringtonine is dissolved in pure ethanol i.e. alkaloid : anhydrous ethanol 1:10 , and crystallized. The crystals are refined by recrystalliation in diethyl ether. Overall yield of Harringtonine is about 0.1% including yield from mixed HH from the subsequent process.

Harringtonine has the following chemical structure:

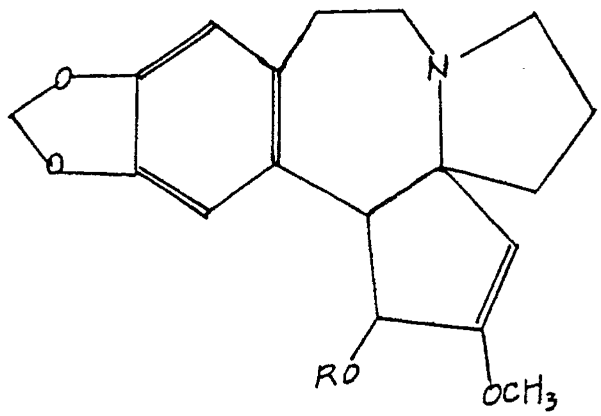

wherein R is

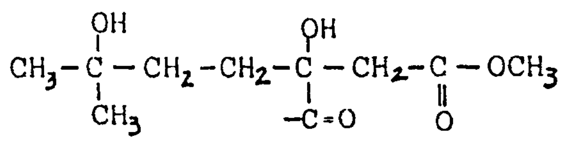

| | |
|---|---|
| melting point: | 135° - 137°C |
| crystal: | colorless |
| infrared spectrum: | 3750, 1660, 1505, 1490, 1050, and 945 $cm^{-1}$. |

ultraviolet spectrum: $\lambda_{peak}^{alcohol}$ nm ($\log\varepsilon$): 244 (3.59), 290 (3.65).

The portion having a pH of 7 and the mother liquors from the foregoing crystallization of Harringtonine are combined and passed through a liquid chromatographic column of diameter to height ratio 1:50 packed with alumina. The column is finally flushed with chloroform and followed by chloroform-methanol of 9:1 mixture. The resulting alkaloids are mixture of HH. The mixed HH is then separated from each other by countercurrent distribution employing chloroform and pH 5 buffer. The first fraction of the countercurrent distribution is Homoharringtonine and the last fraction of the countercurrent distribution is Harringtonine. Homoharringtonine is purified by crystallization in methyl alcohol.
Homoharringtonine has the following chemical structure:

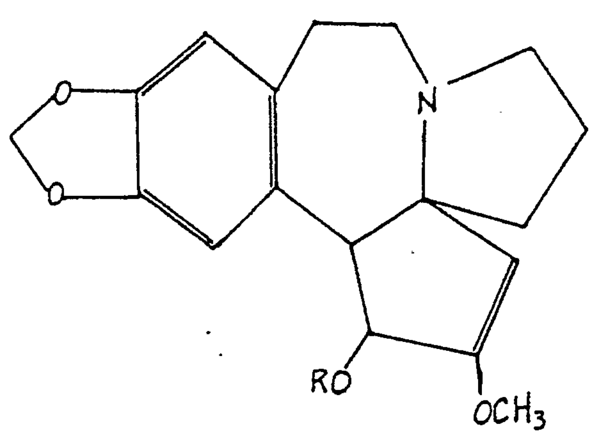

wherein R is

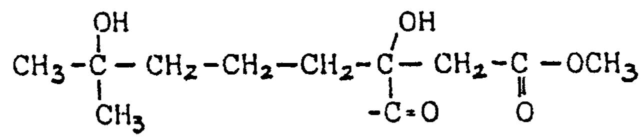

yield: 0.02%

melting point: 144° - 146° C

infrared spectrum: 3500~3400, 1750, 1665, 1030 and 940 $cm^{-1}$.

ultraviolet spectrum: $\lambda_{peak}^{ethanol}$ nm(log$\varepsilon$): 240 (3.55), 290 (3.61)

Example 2. Anethole, (4-Propenylanisol), is extracted from Fennel, or Foeniculum Vulgare Mill. 1 kg of grated Fennel is extracted with 3 l of 95% ethanol at room temperature for 24 hrs. Ethanol is recovered by distillation under reduced pressure from the ethanol extract and a residue containing crude anethole is dissolved in 1 l of distilled water. This aqueous soluton is distilled under reduced pressure whereby Fennel oil is distilled over with steam. Crude Fennel oil is separated from water and extracted with an equal volume of diethyl ether. The ether extract is distilled under reduced pressure thereby recovering the diethyl ether and a residue of Fennel oil respectively. Yield of this crude Fennel oil is about 5.5%.

The resulting Fennel oil is fractionated with reflux on an oil bath. The fraction collected at a distilling temperature from 229-237° C is Anethol.

overall yield: 3.3%

Anethole has the following chemical structure:

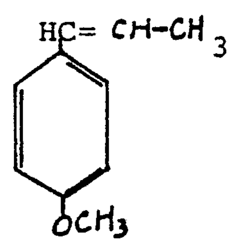

Anethole has a light yellow color.

specific gravity: at 25/25° C 0.983 - 0.987

refractive index at 25°: 1.588 - 1.561

Example 3. Oleanolic acid is extracted from fruits of Ligustrum Lucidum Ait, Hemsleys amabilis Diels or Sweritia meilensis. 1 Kg of the ground fruits of Ligustrum Lucidum Ait. is extracted with 8 l of 95% of ethanol at room temperature for 24 hrs. The residue is separated from filtrate by filtration. The residue is refluxed on water bath with 3 l of 95% ethanol for 6 hrs. and the filtrate collected. The reflux process is repeated once and both filtrates combined. The filtrates are distilled under reduced pressure to recover ethanol and a solid residue. The residue is washed with 1 l of 60° C distilled water. The slurry is filtered and the solids recovered. The solids are dissolved in ethane and the pH adjusted to 11 by adding sodium hydroxide solution. After filtration, HCl is added to the filtrate until the pH is 1. The crystal are separated from the mother liquor which is concentrated to yield more crystals. All the raw crystals are collected, washed with boiling sodium hydroxide solution of pH 11 and filtered. To the crystals distilled water is added and filtered. The crystals are dissolved in ethanol and the pH adjusted to 1 by addition of HCl solution. Purified crystals of Oleanolic acid are obtained, after repeatedly washing the crystals with distilled water till the wash water is neutral.

yield: 0.65%

Oleanolic acid has the following chemical structure:

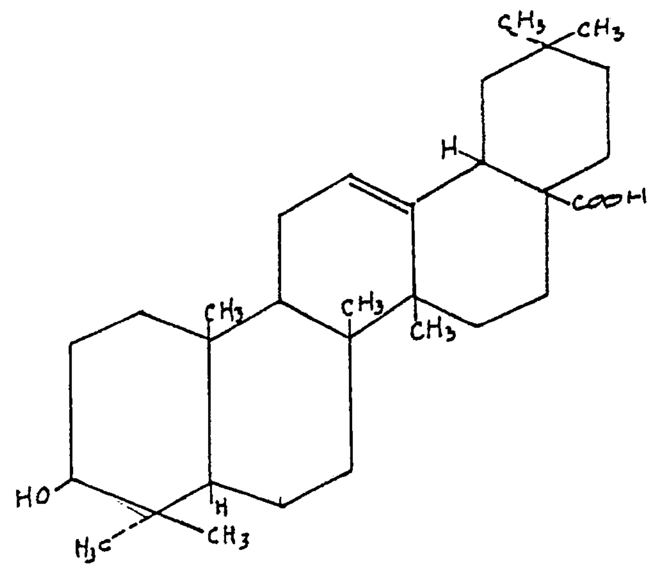

crystal: needle
melting point: 310 °C
specific rotation at 20 °C, +80 ° ($CH_3OH$)

Example 4. Ginsenosides and a method of producing them have been disclosed in copending applications by the same inventor, Yaguang Liu, "Pharmaceutical Composition For Cure And Prevention Of Cardiovascular Disease And Method Of Preparing The Same", US Serial No. 06/730,365 the priority of which has been claimed for EP-A-0 200 169, published on 5.11.86), and "Pharmaceutical Composition Decreasing Side Effects Of Anticancer Chemotherapy, Radiationtherapy And Increasing The Immune Function And Method Of Producing The Same", US Serial No. 06/732,146 (the priority of which has been claimed for EP-A-0 201 784, published on 20.11.86):

1000 g of dried ginseng powder from the roots of Panax guinguefolium L. or Panax ginseng C.A. Meyer is extracted with 200 ml of 95% ethanol at room temperature for 24 hours. The powder is recovered by filtration. Filtrate A is saved and the powder filtercake is refluxed with an additional 2000 ml of 95% ethanol on a steam bath. The mixture is filtered again. Filtrate B is saved and the powder filtercake is refluxed two more times for 6 hours with additional 200 ml batches of 95% ethanol and filtered, providing filtrates C and D. Filtrates A, B, C, and D are combined and distilled at 2,27 kPa (17 mm Hg absolute), whereby ethanol is recovered and a still residue is obtained.

This still residue is dissolved in 500 ml of distilled water. This water solution is extracted five times with 500 ml of a lipophilic solvent, e.g. diethyl ether or petroleum ether, whereby lipids are removed from the solution.

To this acqueous raffinate is added 500 ml of water-saturated n-butanol and the mixture is distilled at 2,27 kPa (17 mm Hg absolute) to dryness, whereby a powder residue is obtained. This powder is dissolved in 500 ml of anhydrous ethanol, and 2000 ml of acetone are added with agitation while a precipitate forms. The precipitate is recovered by filtration and washed twice with acetone and twice with diethyl ether and dried. About 60 g of a white to light yellow powder are recovered, which represents the Ginsenosides.

The composition of a new antileukemia drug effective against granulocytic and monocytic leukemia without cardiotoxicity and damage to hematopoietic organs comprises:

| | | | |
|---|---|---|---|
| Harringtonine | 0.05% - 2.0% | by | weight |
| Homoharringtonine | 0.02% - 1.0% | " | " |
| Anethole | 10.0% - 90.0% | " | " |
| Oleanolic acid | 1.0% - 50.0% | " | " |
| Ginsenosides | 5.0% - 50.0% | " | " |

The dosage of the above for humans would be:

| | |
|---|---|
| Harringtonine | 2.5 mg |
| Homoharringtonine | 1.25 mg |
| Anethol | 300 mg |
| Oleanolic acid | 50 mg |
| Ginsenosides | 100 mg |

The above composition can be manufactured in capsule, tablet or syrup form including ampule for injection according to prevalent art.

Now the invention will be further described with referece to its beneficial effects as illustrated by the following experiment test. It is noted that Avidin-Biotin-Peroxidase complex, known as ABC in immunoperoxidase techniques is used. This technique is widely accepted because of its high sensitivity to the reaction with antigen.

The following is the result of an experiment using the ABC technique in studying the effect of the above composition on the survival rate of chicken myocardial cells.

| | survival rate of chicken myocardial cells (living/total) x 100, % | |
|---|---|---|
| control | 92.0±4.1 | (*10) |
| HH (10u) | 61.2±3.4 | (*20) |
| SAL (HH, 10u+remainder 150u) | 91.4±4.0 | (*20) |
| | $P < 0.001$ | |

* indicates the number of samples

A HH ampule or vial for injection contains

Harringtonine 2.5 mg

Homoharringtonine 1.25 mg

$LD_{50}$ of HH in accordance with the above formula in mice was 4.01 ± 0.28 mg/kg of body weight.

$LD_{50}$ of SAL in accordance with human dosage in mice was 1005 mg/kg. of body weight.

$LD_{50}$ is the median lethal dosage.

## Claims

**1.** A pharmaceutical composition for treating nonlymphatic leukemia, such as granulocytic and monocytic leukemia comprising:

| | | | | |
|---|---|---|---|---|
| Harringtonine | from | 0.05 to 2.0 | wt. | % |
| Homoharringtonine | " | 0.02 to 1.0 | " | " |
| Anethole | " | 10.0 to 90.0 | "" | " |
| Oleanolic Acid | " | 1.0 to 50.0 | " | " |
| Ginsenosides | " | 5.0 to 50.0 | " | " |

**2.** A pharmaceutical composition according to claim 1 in which Harringtonine and Homoharringtonine are produced according to a method comprising:

a) extracting a ground plant selected from Cephalotaxus fortunel Hook, Cephalotaxus sinensis Li, C. hainanensis and C. wilsoniana with 90% ethanol at room temperature for 24 hrs;

b) separating a filtrate A from a filtercake;

c) percolating the filtercake with ethanol and collecting a filtrate B;

d) distilling the combined filtrates A and B, under reduced pressure to recover ethanol and a residue;

e) adjusting the residue to pH 2.5;
f) separating solids from the acidic filtrates;
g) adjusting pH of the filtrates to pH 9.5;
h) extracting five times the alkaline solution of step (g) with chloroform and recovering each chloroform extract; distilling the chloroform extracts to recover the alkaloid;
i) dissolving the alkaloid in citric acid and dividing the solution into three portions and adjusting the pH of the three portions to 7, 8, and 9;
j) distillung portions of pH 8 and 9 together with chloroform to yield the raw alkaloid;
k) purifying said alkaloid by crystallizing in ethanol and recrystallizing in diethyl ether to yield Harringtonine;
l) combining the portion of solution having pH 7 from step (i) and mother liquor from the crystallization of Harringtonine;
m) passing the combined solution through an alumina column of a liquid chromatographic instrument, followed by flushing with chloroform and chloroform-methanol; and
n) separating Harringtonine from Homoharringtonine by countercurrent distribution with chloroform and pH 5 buffer.

**3.** A pharmaceutical composition according to claim 1 or 2 in which Anethole is produced according to a method comprising:
a) extracting grated Fennel with 95% ethanol at room temperature for 24 hrs to yield a filtrate;
b) distilling the filtrate at reduced pressure to recover a residue;
c) dissolving the residue in water and distilling the aqueous solution under reduced pressure whereby Fennel oil is carried over by steam;
d) separating the oil and extracting the same with diethyl ether;
e) distilling the ether extract to yield a residue oil; and fractionating the crude oil and collecting a distillate boiling between 229° and 237° C as pure Anethole.

**4.** A pharmaceutical composition according to one of the claims 1 to 3 in which Oleanolic Acid is produced according to a method comprising:
a) extracting ground fruits of a plant selected from a group consisting of Ligustrum Lucidum Ait, Hemsleys amabilis Diels and Sweritia milensis with 95% ethanol at room temperature for 24 hrs;
b) separating the ground fruits from the filtrate;
c) refluxing twice the ground fruits with fresh 95% ethanol for 6 hrs to yield filtrates;
d) combining filtrates from steps b and c and distilling the filtrates under reduced pressure to yield a solid residue;
e) dissolving the solids in ethanol and adjusting the pH to 11;
f) filtering and adjusting the pH to 1; and
g) crystallizing the Oleanolic Acid, separating and washing the crystal repeatedly with water till the wash water is neutral.

**5.** A pharmaceutical composition according to one of the claims 1 to 4 in which Ginsenosides are produced according to a method comprising:
(a) extracting dried ginseng powder obtained from the roots of Panax quinquefolium L. or Panax ginseng C.A. Meyer with 95% ethanol at room temperature for 24 hrs;
(b) separating a filtrate A;
(c) refluxing the filtercake with 95% ethanol on a steam bath;
(d) separating filtrate B;
(e) refluxing the filtercake two more times for 6 hrs with 95% ethanol;
(f) filtering after each refluxing procedure of (e) to obtain filtrates C and D;
(g) combining filtrates A, B, C and D;
(h) distilling the combined filtrates of (g) under reduced pressure of 2,27 kPa (17 mm Hg absolute) to recover ethanol and to obtain a still residue;
(i) dissolving the still residue in distilled water;
(j) extracting the water solution five times with a lipophilic solvent, e.g. diethyl ether or petroleum ether, to remove lipids from the solution;
(k) adding water saturated n-butanol to the lipid-free solution of (j) and distil at reduced pressure of 2,27 kPa (17 mm Hg absolute) to dryness to obtain a powder residue;
(l) dissolving the powder residue in anhydrous ethanol;

(m) adding acetone with agitation to the ethanolic solution which leads to the formation of a precipitate;
(n) recovering the precipitate by filtration;
(o) washing the recovered precipitate twice with acetone and twice with diethyl ether and drying the precipitate to obtain a light yellow power which represents the Ginsenosides.

**Revendications**

1. Composition pharmaceutique destinée au traitement d'une leucémie non lymphatique, telle que la leucémie granulocytaire et la leucémie monocytaire, comprenant :
0,05 à 2,0 % en poids d'harringtonine
0,02 à 1,0 % en poids d'homoharringtonine
10,0 à 90,0 % en poids d'anéthol
1,0 à 50,0 % en poids d'acide oléanolique
5,0 à 50,0 % en poids de ginsénosides

2. Composition pharmaceutique suivant la revendication 1, dans laquelle l'harringtonine et l'homoharringtonine sont produites suivant un procédé consistant :
a) à soumettre à une extraction une plante broyée choisie entre Cephalotaxus fortunel Hook, Cephalotaxus sinensis Li, C. hainanensis et C. wilsoniana avec de l'éthanol à 90 % à température ambiante pendant 24 heures ;
b) à séparer un filtrat A du gâteau de filtre ;
c) à soumettre le gâteau de filtre à une percolation avec de l'éthanol et à recueillir un filtrat B ;
d) à distiller les filtrats A et B réunis, sous pression réduite, pour séparer l'éthanol et un résidu ;
e) à ajuster le pH du résidu à 2,5 ;
f) à séparer les substances solides des filtrats acides ;
g) à ajuster le pH des filtrats à 9,5 ;
h) à soumettre à cinq extractions la solution alcaline de l'étape (g) avec du chloroforme et à recueillir chaque extrait chloroformique, à distiller les extraits chloroformiques pour séparer l'alcaloïde ;
i) à dissoudre l'alcaloïde dans l'acide citrique et à diviser la solution en trois portions et ajuster le pH des trois portions à 7, 8 et 9 ;
j) à distiller ensemble les portions à pH 8 et 9 avec du chloroforme pour obtenir l'alcaloïde brut ;
k) à purifier ledit alcaloïde par cristallisation dans l'éthanol et recristallisation dans l'éther diéthylique, ce qui donne de l'harringtonine ;
l) à mélanger la portion de solution à pH 7 de l'étape (i) et la liqueur-mère de la cristallisation d'harringtonine ;
m) à faire passer le mélange de solutions à travers une colonne d'alumine d'un appareil de chromatographie en phase liquide, puis à effectuer un balayage rapide avec du chloroforme et un mélange chloroforme-méthanol ; et
n) à séparer l'harringtonine de l'homoharringtonine par distribution à contre-courant avec du chloroforme et un tampon à pH 5.

3. Composition pharmaceutique suivant la revendication 1 ou 2, dans laquelle de l'anéthol est produit suivant un procédé consistant :
a) à soumettre du fenouil râpé à une extraction avec de l'éthanol à 95 % à température ambiante pendant 24 heures, ce qui donne un filtrat ;
b) à distiller le filtrat sous pression réduite pour recueillir un résidu ;
c) à dissoudre le résidu dans l'eau et à distiller la solution aqueuse sous pression réduite, ce qui provoque un entraînement de l'essence de fenouil par la vapeur d'eau ;
d) à séparer l'essence et à soumettre cette essence à une extraction avec de l'éther diéthylique ;
e) à distiller l'extrait éthéré, ce qui donne une huile résiduelle ; et à fractionner l'huile brute et recueillir un distillat bouillant dans la plage de 229° à 237° C, consistant en anéthol pur.

4. Composition pharmaceutique suivant l'une des revendications 1 à 3, dans laquelle de l'acide oléanolique est produit suivant un procédé consistant :
a) à soumettre à une extraction des fruits broyés d'une plante choisie dans le groupe comprenant Ligustrum lucidum Ait, Hemsleys amabilis Diels et Sweritia milensis avec de l'éthanol à 95 % à

température ambiante pendant 24 heures ;
b) à séparer les fruits broyés du filtrat ;
c) à chauffer deux fois au reflux les fruits broyés avec de l'éthanol à 95 % frais pendant 6 heures, ce qui donne des filtrats ;
d) à réunir les filtrats des étapes b et c et distiller les filtrats sous pression réduite, ce qui donne un résidu solide ;
e) à dissoudre les substrats solides dans l'éthanol et ajuster le pH à 11 ;
f) à effectuer une filtration et ajuster le pH à 1 ; et
g) à provoquer la cristallisation de l'acide oléanolique, à séparer et laver les cristaux à plusieurs reprises avec de l'eau jusqu'à la neutralité de l'eau de lavage.

**5.** Composition pharmaceutique suivant l'une des revendications 1 à 4, dans laquelle des ginsénosides sont produits suivant un procédé consistant :
(a) à soumettre à une extraction de la poudre de ginseng séchée obtenue à partir des racines de Panax quinquefolium L. ou de Panax ginseng C.A. Meyer avec de l'éthanol à 95 % à température ambiante pendant 24 heures ;
(b) à séparer un filtrat A ;
(c) à chauffer au reflux le gâteau de filtre avec de l'éthanol à 95 % sur un bain de vapeur d'eau ;
(d) à séparer un filtrat B ;
(e) à soumettre à deux chauffages supplémentaires au reflux le gâteau de filtre pendant 6 heures avec de l'éthanol à 95 % ;
(f) à filtrer, après chaque chauffage au reflux, le produit de l'étape (e), ce qui donne des filtrats C et D ;
(g) à réunir les filtrats A, B, C et D ;
(h) à distiller les filtrats réunis de l'étape (g) sous pression réduite, la pression absolue étant égale à 2,27 kPa (17 mm de Hg), pour recueillir l'éthanol et obtenir un résidu de distillation ;
(i) à dissoudre le résidu de distillation dans de l'eau distillée ;
(j) à soumettre la solution aqueuse à cinq extractions avec un solvant lipophile, par exemple l'éther diéthylique ou l'éther de pétrole, pour éliminer les lipides de la solution ;
(k) à ajouter du n-butanol saturé d'eau à la solution dépourvue de lipides de l'étape (j) et à effectuer une distillation sous pression réduite, la pression absolue étant égale à 2,27 kPa (17 mm de Hg) à siccité, pour obtenir un résidu en poudre ;
(l) à dissoudre le résidu en poudre dans de l'éthanol anhydre ;
(m) à ajouter de l'acétone sous agitation à la solution éthanolique ce qui provoque la formation d'un précipité ;
(n) à séparer le précipité par filtration ;
(o) à laver le précipité recueilli deux fois avec de l'acétone et deux fois avec de l'éther diéthylique et à sécher le précipité pour obtenir une poudre jaune clair qui consiste en ginsénosides.

## Patentansprüche

**1.** Pharmazeutische Zusammensetzung zur Behandlung nichtlymphatischer Leukämie, wie Granulocytenleukämie oder Monocytenleukämie, enthaltend:

| | |
|---|---|
| Harringtonin | 0,05 bis 2,0 Gew.-%, |
| Homoharringtonin | 0,02 bis 1,0 Gew.-%, |
| Anethol | 10,0 bis 90,0 Gew.-%, |
| Oleanolsäure | 1,0 bis 50,0 Gew.-%, |
| Ginsenoside | 5,0 bis 50,0 Gew.-%. |

**2.** Pharmazeutische Zusammensetzung nach Anspruch 1, in welcher Harringtonin und Homoharringtonin gemäß eines Verfahrens mit den folgenden Schritten hergestellt sind:
a) Extrahieren einer gemahlenen Pflanze, ausgewählt aus Cephalotaxus fortunel Hook, Cephalotaxus sinensis Li, Cephalotaxus hainanensis und Cephalotaxus wilsoniana, mit 90 %-igem Ethanol bei

Raumtemperatur für 24 Stunden;
b) Abtrennen eines Filtrats A von einem Filterkuchen;
c) Durchseihen des Filterkuchens mit Ethanol und Sammeln eines Filtrats B;
d) Destilieren der vereinigten Filtrate A und B unter verminderten Druck zur Wiedergewinnung des Ethanols und eines Rückstands;
e) Einstellen des Rückstands auf einen pH-Wert von 2,5;
f) Abtrennen der Feststoffe von den sauren Filtraten;
g) Einstellen des pH-Werts der Filtrate auf 9,5;
h) Fünfmaliges Extrahieren der alkalischen Lösung aus Schritt (g) mit Chloroform und Gewinnung jedes Chloroformextrakts; Destilieren der Chloroformextrakte zur Gewinnung des Alkaloids;
i) Lösen des Alkaloids in Zitronensäure und Aufteilen der Lösung in drei Teile sowie Einstellen des pH-Werts der drei Teile auf 7, 8 und 9;
j) Destilieren der Teile mit den pH-Werten 8 und 9 zusammen mit Chloroform zur Gewinnung des Rohalkaloids;
k) Reinigen des Alkaloids mittels Kristallisierung in Ethanol und Umkristallisierung in Diethylether zur Gewinnung von Harringtonin;
l) Vereinigen des Teils der Lösung mit dem pH-Wert 7 aus dem Schritt (i) und der Mutterlauge aus der Kristallisierung des Harringtonins;
m) Schicken der vereinigten Lösung durch eine Aluminiumoxidsäule eines Flüssigchromatographieinstruments, gefolgt von Spülen mit Chloroform und Chloroform-Methanol; und
n) Abtrennen des Harringtonins vom Homoharringtonin mittels Gegenstromverteilung mit Chloroform und einem Puffer von pH 5.

3. Pharmazeutische Zusammensetzung nach Anspruch 1 oder 2, in welcher Anethol gemäß eines Verfahrens mit den folgenden Schritten hergestellt ist:
a) Extrahieren von geriebenem Fenchel mit 95 %-igem Ethanol bei Raumtemperatur für 24 Stunden zur Gewinnung eines Filtrats;
b) Destilieren des Filtrats bei vermindertem Druck zur Gewinnung eines Rückstands;
c) Lösen des Rückstands in Wasser und Destilieren der wäßrigen Lösung unter vermindertem Druck, wobei Fenchelöl durch Dampf übergeleitet wird;
d) Abtrennen des Öls und Extrahieren desselben mit Diethylether;
e) Destilieren des Etherextrakts zur Gewinnung eines Rückstandsöls; und Fraktioniern des Rohöls sowie Sammeln eines Destilats, welches zwischen 229 und 237° C siedet, als reines Anethol.

4. Pharmazeutische Zusammensetzung nach einem der Ansprüche 1 bis 3, in welcher Oleanolsäure gemäß eines Verfahrens mit den folgenden Schritten hergestellt ist:
a) Extrahieren gemahlener Früchte einer Pflanze ausgewählt aus einer Gruppe bestehend aus Ligustrum Lucidum Ait, Hemsleys amabilis Diels und Sweritia milensis mit 95 %-igem Ethanol bei Raumtemperatur für 24 Stunden;
b) Abtrennen der gemahlenen Früchte von dem Filtrat;
c) Erhitzen der gemahlenen Früchte zweimal unter Rückfluß mit frischem 95 %-igem Ethanol für 6 Stunden zur Gewinnung von Filtraten;
d) Vereinigen der Filtrate aus den Schritten b und c und Destilieren der Filtrate bei vermindertem Druck zur Gewinnung eines festen Rückstands;
e) Lösen der Feststoffe in Ethanol und Einstellen des pH-Werts auf 11;
f) Filtrieren und Einstellen des pH-Werts auf 1; und
g) Kristallisieren der Oleanolsäure, Abtrennen und Waschen des Kristalls wiederholt mit Wasser, bis das Waschwasser neutral ist.

5. Pharmazeutische Zusammensetzung nach einem der Ansprüche 1 bis 4, in welcher die Ginsenoside gemäß eines Verfahrens mit den folgenden Schritten hergestellt sind:
a) Extrahieren von gestrocknetem Ginsengpulver, erhalten aus den Wurzeln von Panax quinquefolium L. oder Panax ginseng C.A. Meyer mit 95 %-igem Ethanol bei Raumtemperatur für 24 Stunden;
b) Abtrennen eines Filtrats A;
c) Erhitzen des Filterkuchens unter Rückfluß mit 95 %-igem Ethanol auf einem Dampfbad;
d) Abtrennen eines Filtrats B;
e) Erhitzen des Filterkuchens zwei weitere Male unter Rückfluß mit 95 %-igem Ethanol für 6 Stunden;

f) Filtrieren nach jedem Rückflußerhitzungsvorgang von Schritt (e), um Filtrate C und D zu erhalten;
g) Vereinigen der Filtrate A, B, C und D;
h) Destilieren der vereinigten Filtrate von Schritt (g) bei vermindertem Druck von 2,27 kPa (17 mm Hg absolut) zur Wiedergewinnung des Ethanols und um einen Destilierrückstand zu erhalten;
i) Lösen des Destilierrückstands in destiliertem Wasser;
j) Fünfmaliges Extrahieren der wäßrigen Lösung mit einem lipophilen Lösungsmittel, beispielsweise Diethylether oder Petrolether, um Lipide aus der Lösung zu entfernen;
k) Zugeben von wassergesättigtem n-Butanol zu der lipidfreien Lösung aus Schritt (j) und Destilieren bei vermindertem Druck von 2,27 kPa (17 mm Hg absolut) bis zur Trockne, um einen pulverigen Rückstand zu erhalten;
l) Lösen des pulverigen Rückstands in wasserfreiem Ethanol;
m) Zugeben von Aceton unter Rühren zu der ethanolischen Lösung, was zur Bildung eines Niederschlags führt;
n) Gewinnung des Niederschlags mittels Filtration;
o) Waschen des gewonnenen Niederschlags zweimal mit Aceton und zweimal mit Diethylether sowie Trocknen des Niederschlags, um ein schwach gelbes Pulver zu erhalten, welches die Ginsenoside darstellt.